# EUROPÄISCHE PATENTSCHRIFT

(11) **EP 2 747 743 B1**
(45) Veröffentlichungstag und Bekanntmachung des Hinweises auf die Patenterteilung: **22.11.2017**
(21) Anmeldenummer: 12733728.5
(22) Anmeldetag: 11.07.2012
(51) Int. Cl.: A61K 8/22, A61K 8/34, A61K 8/81, A61K 8/86, A61K 8/87, A61Q 5/10

(54) **WASSERSTOFFPEROXID-HALTIGE MITTEL MIT VERBESSERTER VISKOSITÄTSEINSTELLUNG**
AGENT COMPRISING HYDROGEN PEROXIDE HAVING IMPROVED VISCOSITY ADJUSTMENT
AGENTS CONTENANT DU PEROXYDE D'HYDROGÈNE À AJUSTEMENT DE VISCOSITÉ AMÉLIORÉ

(30) Priorität: 26.08.2011 DE 102011081607; 26.08.2011 DE 102011081608; 26.08.2011 DE 102011081610; 26.08.2011 DE 102011081611; 26.08.2011 DE 102011081612
(43) Veröffentlichungstag der Anmeldung: 02.07.2014
(73) Patentinhaber: Henkel AG & Co. KGaA, 40589 Düsseldorf (DE)
(72) Erfinder: SCHÖPGENS, Jürgen, 41366 Schwalmtal (DE); JANSSEN, Frank, 41470 Neuss (DE); WADLE, Armin, 40699 Erkrath (DE)
(86) Internationale Anmeldenummer: PCT/EP2012/063564
(87) Internationale Veröffentlichungsnummer: WO 2013/029859

(56) Entgegenhaltungen:
- EP-A1- 0 978 522
- EP-A1- 2 338 470
- WO-A1-02/45674
- WO-A2-02/068488
- WO-A2-2011/082910
- FR-A1- 2 816 210
- US-A1- 2009 081 137
- ANONYMOUS: "Luvigel STAR - Non-ionic, electrolyte tolerant, polyurethane-based associative rheology modifier for personal care applications like skin & sun care, decorative cosmetic or cleansing products", 20090401 , 1. April 2009 (2009-04-01), Seiten 1-18, XP002612053, Gefunden im Internet: URL:http://www.univarusa.com/vwr-inc/tools .nsf/0/6589FB4296753E54882575F5004FDD57/$f ile/Luvigel%20STAR.pdf [gefunden am 2010-11-15]

## Beschreibung

Gegenstand der vorliegenden Anmeldung ist ein wasserstoffperoxid-haltiges Mittel, enthaltend eine Kombination aus mindestens zwei bestimmten Polymeren, zur Anwendung in oxidativen Farbveränderungszubereitungen für keratinische Fasern, insbesondere menschliche Haare, die über eine verbesserte Viskosität verfügt. Diese Viskositätsverbesserung wird durch das Zusammenspiel eines anionischen polymeren Verdickers in Verbindung mit einem bestimmten Polyurethan erzielt.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Diese Mittel sollen neben der gewünschten Färbe und Formleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Daher werden zumeist zweiteilige Färbemittel eingesetzt, aus denen die Anwendungsmischungen erst unmittelbar vor der Anwendung aus einer Farbveränderungszubereitung und einer Oxidationsmittelzubereitung hergestellt werden. Oxidationsfärbemittel zeichnen sich durch hervorragende, lang anhaltende Färbeergebnisse aus.

Auch Aufhellmittel oder Blondiermittel werden üblicherweise unmittelbar vor der Anwendung aus einer wasserstoffperoxid-haltigen Oxidationszubereitung sowie einem Blondierpulver und/oder einer Alkalisierungszubereitung hergestellt.

Oxidationsfärbemittel, Aufhellmittel und Blondiermittel für keratinische Fasern werden im Rahmen der vorliegenden Anmeldung als Farbveränderungszubereitungen bezeichnet.

Um eine optimale Färbeleistung oder Aufhellleistung zu entfalten, benötigen oxidative Farbveränderungszubereitungen in der Regel einen alkalischen pH-Wert, insbesondere zwischen pH 9,0 und pH 11,5. Darüber hinaus beträgt die Anwendungsdauer für ansprechende Färbeergebnisse üblicherweise zwischen 10 und 45 min, für Aufhellergebnisse üblicherweise zwischen 15 und 60 min.

Es ist daher notwendig, dass das anwendungsbereite Farbveränderungszubereitungen so formuliert und konfektioniert ist, dass die Farbveränderungszubereitung sich einerseits gut auf den zu behandelnden keratinischen Fasern verteilen lässt, andererseits jedoch in den zu behandelnden Fasern während der Anwendungszeit verbleibt. Dazu ist es vorteilhaft, wenn das Farbveränderungszubereitung über eine bestimmte Viskosität verfügt, die zwar das Auftragen des Mittels ermöglicht, jedoch das Mittel auch am Ort der Anwendung verbleiben lässt. Andererseits ist es zur Anwendung der Farbveränderungszubereitung wünschenswert, dass sich die Zubereitung durch einfaches Vermischen der Ausgangszubereitungen (Färbezubereitung und Oxidationsmittelzubereitung für Oxidationsfärbemittel bzw. Oxidationsmittelzubereitung und Blondierpulver und/oder Alkalisierungszubereitung für Aufhell- und Blondiermittel) herstellen lässt und darüber hinaus einfach aus dem Mischgefäß ausgebracht und auf die zu behandelnden Fasern aufgetragen werden kann.

Die dazu erforderliche Viskosität kann durch polymere Verdickungsmittel im anwendungsbereiten Farbveränderungszubereitung eingestellt werden, wobei dieses Verdickungsmittel sowohl in der Färbezubereitung bzw. Alkalisierungszubereitung oder der Oxidationsmittelzubereitung enthalten sein kann.

Um eine gute Anmischung zu ermöglichen, ist es vorteilhaft, wenn die Färbezubereitung bzw. Alkalisierungszubereitung und die Oxidationsmittelzubereitung über eine gute Fließfähigkeit verfügen und sich die erhöhte Viskosität der Anwendungsmischung erst nach Vermischen der beiden Komponenten einstellt. Eine Möglichkeit, dieses Ziel zu erreichen, ist der Einsatz von polymeren Verdickungsmitteln, deren verdickende Eigenschaften sich mit dem pH-Wert ändern. Eine Farbveränderungszubereitung besitzt zur Stabilisierung von Oxidationsfarbstoffvorprodukten zumeist einen alkalischen pH-Wert und die Oxidationsmittelzubereitung besitzt zur Stabilisierung des Oxidationsmittels einen sauren pH-Wert, während die Anwendungsmischung einen alkalischen pH-Wert besitzen sollte. Wenn der polymere Verdicker in der sauren Oxidationsmittelzubereitung enthalten ist, ist daher ein anionischer polymerer Verdicker bevorzugt, der bei einem alkalischen pH-Wert zu einer deutlichen Viskositätserhöhung führt.

Als solche anionischen, polymeren Verdicker eignen sich besonders Homo- oder Copolymere von Acrylsäure oder Methacrylsäure. In der Regel sind zur erforderlichen Viskositätseinstellung höhere Mengen, üblicherweise zwischen 2,5 bis 5 Gew.-%, solcher Polymere in der Anwendungsmischung und damit eine entsprechend noch höhere Menge in der Oxidationsmittelzubereitung erforderlich.

Hohe Polymerladungen, insbesondere an anionischen polymeren Verdickungsmitteln, können jedoch bei der Herstellung der Oxidationsmittelzubereitungen zu Problemen führen, da solche erhöhten Einsatzkonzentrationen an Verdickungsmitteln, insbesondere bei geringen pH-Wert-Schwankungen, zu Verstopfungen in den Herstellanlagen und -apparaturen, wie Dosierpumpen und Ventilen führen können. Es ist daher neben der Rohstoffersparnis besonders wünschenswert, Mittel mit verringertem Gehalt an polymeren Verdicker einzusetzen, wenn dabei die Viskosität der Anwendungsmischung nicht beeinträchtigt wird.
Außerdem sollte eine gute Mischbarkeit mit Färbe-, Alkalisierungs- und Blondierzubereitungen gewährleistet sein.

WO 02/45674 A1 beschreibt Mittel zum Färben von Haaren, welche mindestens einen Farbstoff sowie ein assoziatives Polymer enthalten, wobei das assoziative Polymer aus Acrylamid, Dialkyldiallylammonium-Monomeren und Acrylsäure erhalten wird.

In WO 2011/082910 A2 wird ein Zweiphasen-Entwickler offenbart, der zwei voneinander getrennte Phasen umfasst. Die erst Phase enthält ein Oxidationsmittel, und die zweite Phase stellt eine hydrophobe Phase dar. Der Entwickler kann zusätzlich auch ein (Meth)Acrylsäure Homo- oder CoPolymer enthalten.

Aufgabe der vorliegenden Erfindung ist es daher, ein Wasserstoffperoxid-haltiges Mittel für die Anwendung in mehrkomponentigen Farbveränderungszubereitungen für keratinische Fasern zur Verfügung zu stellen, welche über eine gute Mischbarkeit der Teilkomponenten verfügt, jedoch eine ausreichende Viskosität aufweist, so dass die anwendungsbereite Farbveränderungszubereitung sich einerseits gut auftragen lässt, andererseits während der Anwendung am Wirkort verbleibt und nicht aus den Fasern ausfließt. Schließlich soll sich das Mittel dadurch auszeichnen, dass die Menge an polymeren Verdickungsmittel reduziert ist, so dass die oben beschriebenen Probleme während der Herstellung der Mittel minimiert oder eliminiert werden können.

In nicht vorhersehbarer Weise konnte nun gefunden werden, dass durch Kombinationen von verdickenden Polymeren in oxidative Zubereitungen für den Einsatz in farbverändernden Mitteln für keratinische Fasern, insbesondere menschliche Haare, die neben einem anionischen polymeren Verdickungsmittel zusätzlich eine bestimmtes Polyurethan enthält, eine Erhöhung der Viskosität in der anwendungsbereiten Farbveränderungszubereitung ermöglicht wird. Dadurch ist es möglich, die Einsatzmenge an anionischem polymerem Verdickungsmittel zu verringern, ohne Einbussen in der Viskosität der der anwendungsbereiten Farbveränderungszubereitung hinnehmen zu müssen.

Ein erster Gegenstand der vorliegenden Erfindung ist daher ein Mittel, welches in einem kosmetischen geeigneten Träger mindestens Wasserstoffperoxid, mindestens ein Homo- oder Copolymer von Acrylsäure und/oder Methacrylsäure und mindestens ein Polyurethan enthält, und welches dadurch gekennzeichnet ist, dass das Polyurethan ein Polykondensat aus Polyethylenglycol(en), Diisocyanat(en) und gegebenenfalls ethoxylierten Fettalkohol(en) ist, dass das Mittel als Homo- und/oder Copolymer von Acrylsäure und/oder Methacrylsäure mindestens ein Copolymer aus Ethylacrylat sowie Methacrylsäure und/oder Acrylsäure enthält und dass das Polyurethan in einem Gewichtsanteil von 0,05 bis 3,0 Gew.-%, jeweils bezogen auf das Gesamtgewicht des Mittels, enthalten ist.

Unter keratinhaltigen bzw. keratinischen Fasern werden erfindungsgemäß Pelze, Wolle, Federn und insbesondere menschliche Haare verstanden. Obwohl die erfindungsgemäße Verwendung in erster Linie zum Färben und/oder Aufhellen von keratinhaltigen Fasern geeignet sind, steht prinzipiell einer Verwendung auch auf anderen Gebieten nichts entgegen.

Erfindungsgemäße Mittel enthalten die Inhaltsstoffe in einem kosmetischen geeigneten und damit physiologisch verträglichen Träger. Physiologisch verträgliche Träger sind dabei im Rahmen der vorliegenden Anmeldung insbesondere wässrige, wässrig alkoholische und alkoholische Träger. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Mittel.

Als ersten Inhaltsstoff enthält das erfindungsgemäße Mittel Wasserstoffperoxid. Wasserstoffperoxid wird dabei entweder als vorzugsweise wässrige Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon H₂O₂ (n ist eine positive ganze Zahl größer 0), Harnstoffperoxid und Melaminperoxid, eingesetzt. Erfindungsgemäß bevorzugte Mittel enthalten wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt.

Hier sind erfindungsgemäße Mittel besonders bevorzugt, die 0,5 bis 18 Gew.-%, vorzugsweise 1 bis 15 Gew.-%, besonders bevorzugt 2,5 bis 12 Gew.-% und insbesondere 3 bis 9 Gew.-% Wasserstoffperoxid, bezogen auf das Gesamtgewicht des Mittels, (berechnet als 100%iges H₂O₂) enthalten.

Als zweiten wesentlichen Inhaltsstoff enthält das erfindungsgemäße Mittel mindestens ein Polyurethan, welches ein Polykondensat aus Polyethylenglycol(en), Diisocyanat(en) und gegebenenfalls ethoxylierten Fettalkohol(en) ist.

Aufgrund des Fettalkohols enthalten solche erfindungsgemäßen Polyurethane mindestens eine Fettkette in ihrer Struktur und sind daher geeignet, neben hydrophilen Wechselwirkungen aufgrund polarer Struktureinheiten auch hydrophobe Wechselwirkungen untereinander oder mit weiteren Inhaltsstoffen einzugehen und damit zu assoziieren.

Vorzugsweise enthalten die erfindungsgemäßen Polyurethane mindestens zwei hydrophobe Fettketten mit 6 bis 30, bevorzugt 10 bis 24 Kohlenstoffatomen in der Kette, die durch eine hydrophile Struktureinheit voneinander separiert sind. Im Falle von zwei hydrophoben Fettketten bildet sich eine Triblock-Struktur aus, bei der ein zentraler, hydrophiler Abschnitt von zwei lipophilen Struktureinheiten flankiert wird. Es sind jedoch bei mehr als zwei hydrophoben Fettketten in der Struktur auch gleichfalls sternförmige oder gepfropfte Strukturen möglich.

Die hydrophile Struktureinheit der Polyurethane erhält ihren hydrophilen Charakter im Wesentlichen durch die enthaltenen Polyethylenglycol-Einheiten sowie gegebenenfalls den hydrophilen Abschnitt von ethoxylierte Fettalkoholen.

Die Polyurethane sind insbesondere Polykondensate von Polyethylenglycolen mit 50 bis 1000 Einheiten Ethylenglycol (PEG-50 bis PEG-1000), bevorzugt 50 bis 250 Einheiten Ethylenglycol (PEG-50 bis PEG-250).

Als Diisocyanat in den Polykondensaten eignen sich insbesondere Hexamethylendiisocyanat (HDI), Dicyclohexylmethan-4,4'-diisocyanat (SMDI), Isophorondiisocyanat (IPDI), 4,4'-Methylen-bis(phenylisocyanat) (MDI), Diisocyanatotoluol (TDI). Bevorzugt ist Hexamethylendiisocyanat.

Schließlich entstammen die erfindungsgemäßen Polyurethane einer Polykondensation mit gegebenenfalls ethoxylierten Fettalkoholen.

Die Fettalkohole sind dabei gesättigt oder ungesättigt sowie linear oder verzweigt und enthalten dabei insbesondere Fettketten aus 6 bis 30, bevorzugt 10 bis 24 Kohlenstoffatomen in der Kette.

Bevorzugte gegebenenfalls ethoxylierte Fettalkohole sind dabei Decylalkohol, Laurylalkohol, Myristylalkohol, Cetylalkohol, Palmitoleylalkohol, Stearylalkohol, Oleylalkohol, Elaidylalkohol, Linoleylalkohol, Linolenylalkohol, Eicosylalkohol, Gadoleylalkohol, Arachidonalkohol, Behenylalkohol, Erucylalkohol, Brassidylalkohol sowie deren Gemische.

Beispielhaft für Polyurethane, deren Fettalkoholanteil ein Gemisch aus Laurylalkohol und Myristylalkohol ist, ist das Handelsprodukt Elfacos T210 und für Polyurethane, deren Fettalkoholanteil Stearylalkohol ist, ist das Handelsprodukt Elfacos T212 oder das Handelsprodukt Aculyn 46 (Stearylalkohol/PEG-150 bis PEG-180/HDI Polykondensat).

Ein Beispiel für ein Polyurethan mit Decylalkohol als Fettalkoholanteil ist das Handelsprodukt Aculyn 44 (Decylalkohol/PEG-150 bis PEG-180/HDI Polykondensat).

Neben den nicht ethoxylierten Fettalkoholen kann es bevorzugt sein, ethoxylierte Fettalkohole einzusetzen. Der Ethoxylierungsgrad der Fettalkohole kann dabei in drei verschiedene Klassen eingeteilt werden, nämlich niedrig ethoxylierte Fettalkohole mit einem Ethoxylierungsgrad von 1 bis 5 (d.h. 1 bis 5 Mol Ethylenoxid pro Mol Fettalkohol), mittel ethoxylierte Fettalkohole mit einem Ethoxylierungsgrad von 6 bis 30 (d.h. 6 bis 30 Mol Ethylenoxid pro Mol Fettalkohol) und hoch ethoxylierte Fettalkohole mit einem Ethoxylierungsgrad von über 30 (d.h. > 30 Mol Ethylenoxid pro Mol Fettalkohol).

Beispielhaft für Polyurethane mit hoch ethoxylierte Fettalkoholen ist das Handelsprodukt Rheolate FX 1100 (Steareth-100/PEG-136/HDI Polykondensat).

Bevorzugte Polyurethane enthalten mittel ethoxylierte Fettalkohole mit einem mittleren Ethoxylierungsgrad von 6 bis 30, bevorzugt von 6 bis 25. Beispiele solcher ethoxylierter Fettalkohole sind C₁₂-C₁₄-Pareth-8, C₁₂-C₁₄-Pareth-9, C₁₂-C₁₄-Pareth-10, C₁₂-C₁₄-Pareth-11, C₁₂-C₁₄-Pareth-12, C₁₂-C₁₄-Pareth-14, C₁₂-C₁₄-Pareth-15, C₁₂-C₁₄-Pareth-20, C₁₂-C₁₄-Pareth-25, C₁₆-C₁₈-Pareth-8, C₁₆-C₁₈-Pareth-9, C₁₆-C₁₈-Pareth-10, C₁₆-C₁₈-Pareth-11, C₁₆-C₁₈-Pareth-12, C₁₆-C₁₈-Pareth-14, C₁₆-C₁₈-Pareth-15, C₁₆-C₁₈-Pareth-20, C₁₆-C₁₈-Pareth-25, C₁₈-C₂₀-Pareth-8, C₁₈-C₂₀-Pareth-9, C₁₈-C₂₀-Pareth-10, C₁₈-C₂₀-Pareth-11, C₁₈-C₂₀-Pareth-12, C₁₈-C₂₀-Pareth-14, C₁₈-C₂₀-Pareth-15, C₁₈-C₂₀-Pareth-20 sowie C₁₈-C₂₀-Pareth-25.

Dabei kann als Fettalkohol-Komponente im Polyurethan ein Gemisch aus Fettalkoholen unterschiedlicher Kettlänge und unabhängig davon unterschiedlichen mittleren Ethoxylierungsgrad einkondensiert sein.

Eine Ausführungsform des ersten Erfindungsgegenstands ist ein Mittel, welches dadurch gekennzeichnet ist, dass es als Polyurethan ein Polykondensat aus Polyethylenglycol mit 50 bis 250 Einheiten Ethylenglycol (PEG-50 bis PEG-250), einem Diisocyanat und ethoxylierten C₁₂-C₂₀-Fettalkoholen mit einem Ethoxylierungsgrad von 6 bis 25 enthält.

Besonders bevorzugt ist das Polyurethan mit der INCl-Bezeichnung Polyurethan-39, welches ein Polykondensat aus PEG-140, Hexamethylendiisocyanat und einem Gemisch aus C₁₂-C₁₄-Pareth-1 0, C₁₆-C₁₈-Pareth-11 und C₁₈-C₂₀-Pareth-11 darstellt, und welches beispielsweise von der Firma BASF SE unter der Handelsbezeichnung Luvigel Star vertrieben wird.

Eine bevorzugte Ausführungsform des ersten Erfindungsgegenstands ist daher ein Mittel, welches dadurch gekennzeichnet ist, dass das Polyurethan ein Polykondensat aus PEG-140, Hexamethylendiisocyanat (HDI) und einem Gemisch aus C₁₂-C₁₄-Pareth-10, C₁₆-C₁₈-Pareth-11 und C₁₈-C₂₀-Pareth-11 ist.

Ein erfindungsgemäßes Mittel enthält das Polyurethan in einem Gewichtsanteil von 0,05 bis 3,0 Gew.-% und insbesondere von 0,1 bis 2,5 Gew.-%, jeweils bezogen auf das Gesamtgewicht des Mittels.

Als dritten wesentlichen Inhaltsstoff enthält das Mittel des ersten Erfindungsgegenstands mindestens ein Homo- oder Copolymer von Acrylsäure und/oder Methacrylsäure, wobei das Mittel als Homo- und/oder Copolymer von Acrylsäure und/oder Methacrylsäure mindestens ein Copolymer aus Ethylacrylat sowie Methacrylsäure und/oder Acrylsäure enthält.

Da das Mittel zur Stabilisierung des Wasserstoffperoxids üblicherweise einen sauren pH-Wert besitzt, die Anwendungsmischung jedoch einen alkalischen pH-Wert aufweist, unterliegt das Homo- oder Copolymer von Acrylsäure und/oder Methacrylsäure einer pH-Wertveränderung, durch die die Carbonsäuregruppen von Acrylsäure- oder Methacrylsäure-Einheiten deprotoniert werden und durch diese Ionisierung eine Gelbildung und damit eine Viskositätserhöhung einsetzt.

Neben Acrylsäure und Methacrylsäure sind Crotonsäure, Itaconsäure, Maleinsäureanhydrid und 2-Acrylamido-2-methylpropansulfonsäure weitere Beispiele für anionische Monomere, aus denen die polymeren anionischen Verdickungsmittel bestehen können. Dabei können die sauren Gruppen ganz oder teilweise als Natrium-, Kalium-, Ammonium-, Mono- oder Triethanolammonium-Salz vorliegen.

Bevorzugt kann die erfindungsgemäße Oxidationsmittelzubereitung zusätzlich mindestens ein anionisches Acrylsäure- und/oder Methacrylsäure-Polymerisat oder -Copolymerisat enthalten. Bevorzugte Polymerisate dieser Art sind:
- Mischpolymerisate aus 50 bis 75 Gew.-% Ethylacrylat, 25 bis 35 Gew.-% Acrylsäure und 0 bis 25 Gew.-% anderer Comonomeren bekannt. Geeignete Dispersionen dieser Art sind im Handel erhältlich, z.B. unter der Handelsbezeichnung Latekoll® D (BASF).
- Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Bevorzugt ist dabei die Kombination aus Methacrylsäure und Ethylacrylat sowie gegebenenfalls vernetzenden, multifunktionalen Monomeren. Ein bevorzugtes Handelsprodukt dafür ist beispielsweise Aculyn® 33 bzw. 33A der Firma Rohm & Haas.

Erfindungsgemäß ist daher ein Mittel, welches dadurch gekennzeichnet ist, dass das Mittel als Homo- und/oder Copolymer von Acrylsäure und/oder Methacrylsäure mindestens ein Copolymer aus Ethylacrylat sowie Methacrylsäure und/oder Acrylsäure enthält.

Durch die erfindungsgemäße Polymerkombination lässt sich der Anteil an Homo- oder Copolymeren im erfindungsgemäßen Mittel ohne Einbussen der Anwendungsviskosität der Farbveränderungszubereitung gering halten, bevorzugt unter 2 Gew.-%, bezogen auf das Mittel.

Eine Ausführungsform des ersten Erfindungsgegenstands ist daher ein Mittel, welches dadurch gekennzeichnet ist, dass das/die Homo- und/oder Copolymer(e) von Acrylsäure und/oder Methacrylsäure in einem Gewichtsanteil von 0,001 bis 2,0 Gew.-%, bevorzugt 0,005 bis 1,0 Gew.-% und insbesondere von 0,01 bis 0,5 Gew.-%, jeweils bezogen auf das Gesamtgewicht des Mittels, enthalten ist.

Weiterhin hat es sich als vorteilhaft erweisen, wenn die Mittel mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbildner sind Stoffe, die Metallionen komplexieren können. Bevorzugte Komplexbildner sind sogenannte Chelatkomplexbildner. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Ethylendiamindibernsteinsäure (EDDS), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-atomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/ oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Zur weiteren Stabilisierung des Wasserstoffperoxids und damit verbesserten Lagerfähigkeit weisen die erfindungsgemäßen Mittel bevorzugt einen sauren pH-Wert, bevorzugt zwischen pH 2 und pH 5, insbesondere bevorzugt zwischen pH 3,5 bis pH 4,5, auf. Zur Einstellung des pH-Werts sind dem Fachmann in der Kosmetik gängige Acidifizierungs- und Alkalisierungsmittel als pH-Stellmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Die erfindungsgemäßen Mittel des ersten Erfindungsgegenstands werden mit einer weiteren Zubereitung zur anwendungsbereiten Farbveränderungszubereitung vermischt. Diese anwendungsbereite Farbveränderungszubereitung besitzt zur Aktivierung des Wasserstoffperoxids genauso wie zur Quellung der keratinischen Fasern und damit zum erleichterten Eindringen der Wirkstoffe bevorzugt einen alkalischen pH-Wert, bevorzugt einen pH-Wert im Bereich von 8 bis 12. Bei den pH-Werten im Sinne der vorliegenden Erfindung handelt es sich um pH-Werte, die bei einer Temperatur von 22 °C gemessen wurden.

Die beigemischte Zubereitung enthält daher bevorzugt mindestens ein Alkalisierungsmittel, um den sauren pH-Wert des Mittels des ersten Erfindungsgegenstands auszugleichen.

Es hat sich weiterhin als vorteilhaft herausgestellt, wenn die beigemischte Zubereitung zusätzlich
(i) mindestens einen Fettalkohol und/oder
(ii) mindestens ein anionisches, amphoteres und/oder zwitterionisches Tensid und/oder
(iii) mindestens eine Aminosäure und/oder
(iv) mindestens einen ethoxylierten Fettalkohol und/oder
(v) mindestens ein kationisches und/oder amphoteres Polymer
enthält.

Ein weiterer Gegenstand der vorliegenden Anmeldung ist daher eine Zubereitung zur Farbveränderung keratinischer Fasern, insbesondere menschlicher Haare, welche dadurch gekennzeichnet ist, dass die Zubereitung unmittelbar vor der Anwendung durch Vermischen von mindestens einem Mittel des ersten Erfindungsgegenstands und mindestens einer Alkalisierungszubereitung, enthaltend in einem kosmetisch geeigneten Träger mindestens ein Alkalisierungsmittel und zusätzlich
(i) mindestens einen Fettalkohol und/oder
(ii) mindestens ein anionisches, amphoteres und/oder zwitterionisches Tensid und/oder
(iii) mindestens eine Aminosäure und/oder
(iv) mindestens einen ethoxylierten Fettalkohol und/oder
(v) mindestens ein kationisches und/oder amphoteres Polymer,
hergestellt wird.

Die Alkalisierungsmittel der Alkalisierungs- zubereitung werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Erfindungsgemäß bevorzugte Alkanolamine werden ausgewählt aus der Gruppe Triethanolamin, 2-Aminoethan-1-ol (Monoethanolamin), 2-Amino-2-methylpropan-1-ol und 2-Amino-2-methyl-propan-1,3-diol. Ein besonders bevorzugtes Alkanolamin ist Monoethanolamin. Geeignete basische Aminsäuren sind beispielsweise Lysin, Arginin und Ornithin. Erfindungsgemäße, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Ammoniumcarbonat, Natriumcarbonat und Kaliumcarbonat.
In einer Ausführungsform enthält die Alkalisierungszubereitung zusätzlich mindestens einen Fettalkohol. Fettalkohole im Sinne der vorliegenden Anmeldung sind dabei gesättigte oder ungesättigte sowie lineare oder verzweigte primäre Alkohole, die insbesondere Fettketten aus 6 bis 30, bevorzugt 10 bis 24 Kohlenstoffatomen in der Kette enthalten.

Bevorzugte lineare Fettalkohole sind dabei Decylalkohol, Laurylalkohol, Myristylalkohol, Cetylalkohol, Palmitoleylalkohol, Stearylalkohol, Oleylalkohol, Elaidylalkohol, Linoleylalkohol, Linolenylalkohol, Eicosylalkohol, Gadoleylalkohol, Arachidonalkohol, Behenylalkohol, Erucylalkohol, Brassidylalkohol sowie deren Gemische, die gegebenenfalls bei technischen Herstellung oder Gewinnung anfallen, wie Cetearylalkohol, Cocosfettalkohol oder Talgfettalkohol.

Im Rahmen der vorliegenden Anmeldung sind auch verzweigte Fettalkohole bevorzugt. Zu erfindungsgemäß einsetzbaren verzweigten Fettalkoholen zählen insbesondere die sogenannten Guerbert-Alkohole, die aus primären Alkoholen durch die Guerbert-Reaktion erhältlich sind. Beispiele solcher Guerbert-Alkohole sind (2-)Ethyl-hexylalkohol, (2-)Octyl-dodecylalkohol oder (2-)-Hexyl-decylalkohol.

Die erfindungsgemäße Alkalisierungszubereitung enthält Fettalkohole bevorzugt in einem Anteil von 0,5 bis 20 Gew.-%, bevorzugt 1 bis 15 Gew.-%, insbesondere 1,5 bis 12 Gew.-%, jeweils bezogen auf das Gesamtgewicht der Alkalisierungszubereitung.

In einer weiteren Ausführungsform enthält die Alkalisierungszubereitung bevorzugt zusätzlich mindestens ein anionisches, amphoteres und/oder zwitterionisches Tensid.

Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate. Erfindungsgemäß bevorzugte anionische Tenside sind Seifen, Alkylsulfate, Alkylethersulfate und Ethercarbonsäuren.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Beispiele solcher zwitterionischen Tenside sind die sogenannten Betaine wie die N-Alkyl-N,N-dimethylammonium-glycinate, beispielsweise das Ko-kosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Übliche amphotere Tenside sind N-Alkylglycine, N- Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Beispielhafte amphotere Tenside sind N-Kokosalkylaminopropionat, Kokosacylaminoethylaminopropionat, C₁₂-C₁₈-Acylsarcosin und insbesondere das unter der INCI-Bezeichnung bekannte Disodium Cocoamphodipropionate.

Erfindungsgemäß bevorzugt anionische, amphotere und/oder zwitterionische Tenside sind dabei ausgewählt aus Sodium Laureth Sulfate, Sodium Lauryl Sulfate, Sodium Myreth Sulfate, Sodium Cetearyl Sulfate, Sodium Ceteareth Sulfate, Potassium Oleate, Potassium Isostearate, Potassium Myristate, Sodium Laureth-6 Carboxylate, Cocamidopropyl Betaine, Disodium Cocoamphodipropionate sowie deren Gemischen.

Die erfindungsgemäße Alkalisierungszubereitung enthält anionische, amphotere und/oder zwitterionische Tenside bevorzugt in einem Gesamtanteil von 0,1 bis 8,0 Gew.-%, bevorzugt 0,2 bis 5,0 Gew.-%, insbesondere 0,5 bis 3,0 Gew.-%, jeweils bezogen auf das Gesamtgewicht der Alkalisierungszubereitung.

In einer weiteren Ausführungsform enthält die Alkalisierungszubereitung bevorzugt zusätzlich mindestens eine Aminosäure.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

α-Aminocarbonsäuren enthalten üblicherweise mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Erfindungsgemäß bevorzugte Aminosäuren sind Arginin, Serin, Lysin, Glycin, Tyrosin, Prolin, Glutamin, Cystein und Histidin sowie deren Mischungen, besonders bevorzugt sind Arginin, Serin, und/oder Glycin.

Die Aminosäuren können den erfindungsgemäßen Alkalisierungszubereitungen bevorzugt in freier Form zugegeben werden. In einer Reihe von Fällen ist es jedoch auch vorteilhaft, die Aminosäuren in Salzform einzusetzen. Bevorzugte Salze sind insbesondere Hydrochloride, Hydrobromide und Sulfate.

Die Aminosäure(n) und/oder deren Salze ist/sind in den Alkalisierungszubereitungen bevorzugt in Mengen von 0,1 bis 5 Gew.-%, insbesondere von 0,5 bis 3 Gew.-%, bezogen auf das Gesamtgewicht der Alkalisierungszubereitung, enthalten.

In einer weiteren Ausführungsform enthält die Alkalisierungszubereitung bevorzugt zusätzlich mindestens einen ethoxylierten Fettalkohol. Die Fettalkohole sind dabei bevorzugt gesättigt oder ungesättigt sowie linear oder verzweigt und enthalten dabei insbesondere Fettketten aus 6 bis 30, bevorzugt 10 bis 24 Kohlenstoffatomen in der Kette.

Bevorzugte ethoxylierte Fettalkohole sind dabei ausgewählt aus ethoxylierten, linearen Fettalkoholen, bevorzugt einer Kettenlänge mit 8 bis 22 Kohlenstoffatomen. Unter einem ethoxylierten Fettalkohol wird im Sinne der Erfindung ein Anlagerungsprodukt von Ethylenoxid an einen Fettalkohol verstanden, wobei der Ethoxylierungsgrad die Molmenge Ethylenoxid (EO) angibt, die durchschnittlich pro Mol Fettalkohol angelagert wurde.

Bevorzugte ethoxylierte Fettalkohole sind Ethylenoxid-Anlagerungsprodukte an Caprinalkohol, Decylalkohol, Laurylalkohol, Isotridecylalkohol, Myristylalkohol, Cetylalkohol, Palmoleylalkohol, Stearylalkohol, Oleylalkohol, Elaidylalkohol, Petroselinylalkohol, Linolylalkohol, Linolenylalkohol, Elaeostearylalkohol, Eicosylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Besonders bevorzugt sind Anlagerungsprodukte an technische Fettalkohole bzw. deren Mischungen mit 12 bis 18 Kohlenstoffatomen wie beispielsweise Kokos-, Palm-, Palmkern- oder Talgfettalkohol, insbesondere Kokos- und/ oder Talgfettalkohol.

Je nach Herstellungsmethode fallen die erfindungsgemäßen ethoxylierten Fettalkohole als ein Gemisch mit einer unterschiedlichen Ethoxylierungsgradverteilung an. Im Sinne der Erfindung werden diese Emulgatoren daher nach dem durchschnittlichen Ethoxylierungsgrad gekennzeichnet. Dieser ist üblicherweise als Ziffer hinter dem Fettalkohol-Suffix "eth-" in der INCI-Bezeichnung erkennbar.

Die ethoxylierten Fettalkohole lassen dabei je nach durchschnittlichen Ethoxylierungsgrad in drei verschiedene Klassen einteilen, nämlich niedrig ethoxylierte Fettalkohole mit einem Ethoxylierungsgrad von 1 bis 5 (d.h. 1 bis 5 Mol Ethylenoxid pro Mol Fettalkohol), mittel ethoxylierte Fettalkohole mit einem Ethoxylierungsgrad von 6 bis 30 (d.h. 6 bis 30 Mol Ethylenoxid pro Mol Fettalkohol) und hoch ethoxylierte Fettalkohole mit einem Ethoxylierungsgrad von über 30 (d.h. > 30 Mol Ethylenoxid pro Mol Fettalkohol).

Bevorzugte ethoxylierte Fettalkohole mit einem niedrigen, durchschnittlichen Ethoxylierungsgrad sind beispielsweise die Handelsprodukte Laureth-2 (Dehydol LS 2, Arlypon FT 90, Firma BASF), Ceteareth-2 (Lowenol C 279, Firma Lowenstein), Ceteth-2 (Nikkol BC 2, Firma Nikko Chemicals), Steareth-2 (Lipocol S 2, Firma Lipo Chemicals), Laureth-3 (Marlipal 24 30, Firma Sasol), Cetearth-3 (Hostacerin T 3, Firma Clariant), Laureth-4 (Emulgen 104 P, Firma KAO; Nikkol BL 4.2, Firma Nikko Chemicals).

Bevorzugte ethoxylierte Fettalkohole mit einem mittleren, durchschnittlichen Ethoxylierungsgrad sind beispielsweise die Handelsprodukte Ceteareth-6 (Eumulgin CS 6, Firma BASF), Laureth-7 (Marlipal 24 70, Firma Sasol), Ceteth-7 (Nikkol BC 7, Firma Nikko Chemicals), Laureth-9 (Marlipal 24 90, Firma Sasol; Nikkol BL 9, Firma Nikko Chemicals), Laureth-10, Ceteareth-12 (Eumulgin B 1, Firma Cognis), Ceteareth-13 (Emulgen 220, Firma KAO), Ceteth-15 (Nikkol BC 15 TX, Firma Nikko Chemicals Co., Ltd.), Laneth-15 (Polychol 15, Firma Croda), Ceteareth-15 (Eumulgin CS 15, Firma Cognis); Laneth-16 (und) Ceteth-16 (und) Oleth-16 (und) Steareth-16 (als Gemisch vertrieben unter dem Handelsnamen Solulan 16, Firma Noveon); Oleth-20 (Ritoleth 20, Firma Rita Corp.), Ceteth-20 (Brij 58 SP, Firma Uniqema; Lipocol C 20, Firma Lipo Chemicals Inc.), Ceteareth-20 (Surfac JH 200, Firma Surfachem; Eumulgin B 2, Firma Cognis), Laneth-20 (Polychol 20, Firma Croda); Steareth-21 (Brij 721 P, Firma Uniqema; Eumulgin S 21, Firma Cognis); Ceteareth-23 (Mergital C 23, Firma Cognis), Laureth-23 (Canasol BJ 35, Firma Canamax); Ceteareth-25 (Cremophor A 25, Firma BASF); Ceteareth-27 (Plurafac A 38, Firma BASF); Ceteareth-30 (Lipocol SC 30, Firma Lipo Chemicals; Eumulgin B 3, Firma Cognis).

Bevorzugte ethoxylierte Fettalkohole mit einem hohen, durchschnittlichen Ethoxylierungsgrad sind beispielsweise die Handelsprodukte: Ceteth-40 (Nikkol BC 40 TX, Firma Nikko Chemicals Co., Ltd.), Laneth-40 (Polychol 40, Firma Croda); Oeth-50 (Nikkol BO 50 V, Firma Nikko Chemicals Co., Ltd.), Ceteareth-50 (Genapol T 500, Firma Clariant; Mergital CS 50, Firma Cognis), Ceteareth-60 (Findet 1618 A 72, Firma KAO Corp.), Ceteareth-80 (Lutensol AT 80, Firma BASF), Ceteth-150 (Nikkol BC 150, Firma Nikko Chemicals). Besonders bevorzugt ist Ceteareth-50.
Erfindungsgemäß besonders bevorzugte ethoxylierte Fettalkohole sind ausgewählt aus Ceteareth-12, Ceteareth-20, Ceteareth-30 und/oder Ceteareth-50.

Die Alkalisierungszubereitungen enthalten ethoxylierte Fettalkohole bevorzugt in einem Anteil von 0,1 bis 10 Gew.-%, insbesondere von 0,2 bis 5 Gew.-%, bezogen auf das Gesamtgewicht der Alkalisierungszubereitung.

In einer weiteren Ausführungsform enthält die Alkalisierungszubereitung bevorzugt zusätzlich mindestens ein amphoteres und/oder kationisches Polymer.

Als kationische und/oder amphotere Polymere sind solche Polymere zu verstehen, die wenigstens ein quartäres Stickstoffatom, beispielsweise in Form einer Ammoniumgruppe, enthalten. Geeignete kationische und/oder amphotere Polymere sind beispielsweise
- quaternisierte Cellulose-Derivate, wie sie unter den Bezeichnungen Celquat und Polymer JR, bevorzugt Celquat H 100, Celquat L 200 und Polymer JR 400, im Handel erhältlich sind;
- Copolymere des Vinylpyrrolidinons mit quaternierten Derivaten des Dialkylaminoacrylats und - methacrylats, wie beispielsweise mit Diethylsulfat quaternierte Vinylpyrrolidinon-Dimethylaminomethacrylat-Copolymere, die unter den Bezeichnungen Gafquat 734 und Gafquat 755 im Handel (INCl-Bezeichnung Polyquternium-11) erhältlich sind;
- Vinylpyrrolidon-Methoimidazoliniumchlorid-Copolymere, wie sie unter der Bezeichnung Luviquat angeboten werden;
- quaternierter Polyvinylalkohol,
- quaternierte Harnstoff- oder Urethan-haltige Polymere, wie Polymere der INCl-Bezeichnung Polyquaternium-2 bzw. Polyquaternium-27,
- quaternierte Amid-haltige Polymere, wie Polymere der INCI-Bezeichnung Polyquaternium-17 oder Polyquaternium-18 sowie
- Homo- oder Copolymere von Diallyldimethylammoniumchlorid.

Ein erfindungsgemäß bevorzugtes kationisches und/oder amphoteres Polymer ist mindestens Homo- oder Copolymer von Diallyldimethylammoniumchlorid.

In den Copolymeren von Diallyldimethylammoniumchlorid können bevorzugt zusätzlich anionische oder nichtionische Monomere eingesetzt werden, wie beispielsweise Acrylsäure, Methacrylsäure, Alkylester von Acrylsäure oder von Methacrylsäure, Acrylsäureamid, Vinylimidazol, Vinylpyrrolidinon oder Vinylacetat.
In einer besonders bevorzugten Ausführungsform ist das Homo- oder Copolymer von Diallyldimethylammoniumchlorid ausgewählt aus Diallyldimethylammoniumchlorid-Homopolymer (Handelsname Merquat 100; Polyquaternium-6), Diallyldimethylammoniumchlorid-Acrylsäureamid-Copolymer (Handelsname Merquat 550; Potyquaternium-7), Diallyldimethylammoniumchlorid-Acrylsäure-Copolymer (Handelsname Merquat 280 bzw. Merquat 281; Polyquaternium-22), Diallyldimethylammoniumchlorid-Acrylsäureamid-Acrylsäure-Copolymer (Handelsname Merquat Plus 3330; Polyquaternium-39) und/oder Diallyldimethylammoniumchlorid-Vinylimidazol-Vinylpyrrolidinon-Copolymer (Handelsname Luviquat Sensation; Polyquaternium-87).

In einer weiteren bevorzugten Ausführungsform ist das kationische und/oder amphotere Polymer ein Homo- oder Copolymer von Estern oder Amiden der Acrylsäure- oder Methacrylsäure als Monomere, welche eine kationische Ladung in der Ester- bzw. Amid-Seitenkette tragen. Beispiele solcher Monomeren sind Acrylamidopropyltrimonium chloride, N,N-Dimethyl-N-1-[3-(2-methyl-1-oxo-2-propenyl)amino]propyl]-1-dodecanaminium chloride (Methacrylamidopropyl-dodecyldimonium Chloride), 2-(Trimethylammonio)ethyl methacrylate chloride, N,N,N-Trimethyl-N-3-(2-methyl-1-oxo-2-propenyl)amino]-1-propanaminium chloride (Methacrylamidopropyltrimonium Chloride) und 2-(N,N-Dimethyl-N-ethylammonio)ethyl methacrylate Ethylsulfate.

In einer besonders bevorzugten Ausführungsform ist das Homo- oder Copolymer von Estern oder Amiden der Acrylsäure- oder Methacrylsäure ausgewählt aus Polyquaternium-11 (Handelsnamen Gafquat 440, Gafquat 734, Gafquat 755, Luviquat PQ-11 PN), Polyquaternium-28 (Handelsname Gafquat HS 100), Polyquaternium-37 (Handelsname Synthalen), Polyquaternium-55 (Handelsname Styleze W 20 / W 10), Polyquaternium-69 (Handelsname Aquastyle 200) oder Acrylamidopropyltrimonium Chloride/Acrylates Copolymer (Handelsname Produkt W 37194).

In einer besonders bevorzugten Ausführungsform enthält das Alkalisierungsmittel mindestens ein kationisches und/oder amphoteres Polymer, ausgewählt aus Polyquaternium-2, Polyquaternium-4, Polyquaternium-6, Polyquaternium-7, Polyquaternium-10, Polyquaternium-11, Polyquaternium-16, Polyquaternium-18, Polyquaternium-22, Polyquaternium-24, Polyquaternium-28, Polyquaternium-32, Polyquaternium-37, Polyquaternium-39, Polyquaternium-44, Polyquaternium-46, Polyquaternium-55, Polyquaternium-59, Polyquaternium-67, Polyquaternium-68, Polyquaternium-69, Polyquaternium-72, Polyquaternium-87, Acrylamidopropyltrimonium Chloride/Acrylates Copolymer und deren Mischungen.

Es ist bevorzugt, wenn das kationische und/oder amphotere Polymer in einem Anteil von 0,1 bis 5 Gew.-%, bevorzugt 0,2 bis 3 Gew.-%, jeweils bezogen auf das Gesamtgewicht der Alkalisierungszubereitung, in der erfindungsgemäßen Alkalisierungszubereitung enthalten ist.

In einer besonders bevorzugten Ausführungsform enthält die Alkalisierungszubereitung zusätzlich mindestens einen Fettalkohol und weiterhin mindestens ein anionisches und/oder zwitterionisches und/oder amphoteres Tensid und/oder mindestens ein amphoteres und/oder kationisches Polymer und/oder mindestens einen ethoxylierten Fettalkohol und/oder mindestens eine Aminosäure.

In einer besonders bevorzugten Ausführungsform enthält die Alkalisierungszubereitung zusätzlich mindestens ein anionisches und/oder zwitterionisches und/oder amphoteres Tensid und weiterhin mindestens einen Fettalkohol und/oder mindestens ein amphoteres und/oder kationisches Polymer und/oder mindestens einen ethoxylierten Fettalkohol und/oder mindestens eine Aminosäure.

In einer besonders bevorzugten Ausführungsform enthält die Alkalisierungszubereitung zusätzlich mindestens ein amphoteres und/oder kationisches Polymer und weiterhin mindestens ein anionisches und/oder zwitterionisches und/oder amphoteres Tensid und/oder mindestens einen Fettalkohol und/oder mindestens einen ethoxylierten Fettalkohol und/oder mindestens eine Aminosäure.

In einer besonders bevorzugten Ausführungsform enthält die Alkalisierungszubereitung zusätzlich mindestens einen ethoxylierten Fettalkohol und weiterhin mindestens ein anionisches und/oder zwitterionisches und/oder amphoteres Tensid und/oder mindestens ein amphoteres und/oder kationisches Polymer und/oder mindestens einen Fettalkohol und/oder mindestens eine Aminosäure.

In einer besonders bevorzugten Ausführungsform enthält die Alkalisierungszubereitung zusätzlich mindestens eine Aminosäure und weiterhin mindestens ein anionisches und/oder zwitterionisches und/oder amphoteres Tensid und/oder mindestens ein amphoteres und/oder kationisches Polymer und/oder mindestens einen ethoxylierten Fettalkohol und/oder mindestens einen Fettalkohol.

Ganz besonders bevorzugt sind Alkalisierungszubereitungen, die zusätzlich mindestens einen Fettalkohol und mindestens ein anionisches Tensid und mindestens ein amphoteres Polymer und mindestens einen ethoxylierten Fettalkohol und mindestens eine Aminosäure enthalten.

Ganz besonders bevorzugt sind Alkalisierungszubereitungen, die zusätzlich mindestens einen Fettalkohol und mindestens ein zwitterionisches Tensid und mindestens ein amphoteres Polymer und mindestens einen ethoxylierten Fettalkohol und mindestens eine Aminosäure enthalten.

Ganz besonders bevorzugt sind Alkalisierungszubereitungen, die zusätzlich mindestens einen Fettalkohol und mindestens ein amphoteres Tensid und mindestens ein amphoteres Polymer und mindestens einen ethoxylierten Fettalkohol und mindestens eine Aminosäure enthalten.

Ganz besonders bevorzugt sind Alkalisierungszubereitungen, die zusätzlich mindestens einen Fettalkohol und mindestens ein anionisches Tensid und mindestens ein kationisches Polymer und mindestens einen ethoxylierten Fettalkohol und mindestens eine Aminosäure enthalten.

Ganz besonders bevorzugt sind Alkalisierungszubereitungen, die zusätzlich mindestens einen Fettalkohol und mindestens ein zwitterionisches Tensid und mindestens ein kationisches Polymer und mindestens einen ethoxylierten Fettalkohol und mindestens eine Aminosäure enthalten.

Ganz besonders bevorzugt sind Alkalisierungszubereitungen, die zusätzlich mindestens einen Fettalkohol und mindestens ein amphoteres Tensid und mindestens ein kationisches Polymer und mindestens einen ethoxylierten Fettalkohol und mindestens eine Aminosäure enthalten.

Die Zubereitung zur Farbveränderung keratinischer Fasern wird dabei in einem bestimmten Gewichts- oder Volumen-Verhältnis von einem Mittel des ersten Erfindungsgegenstands und mindestens einer Alkalisierungszubereitung hergestellt. Bevorzugt sind dabei Farbveränderungszubereitungen, bei denen das Gewichtsverhältnis aus wasserstoffperoxid-haltigen Mittel des ersten Erfindungsgegenstands und Alkalisierungszubereitung ein Verhältnis von 4 : 1 bis 1 : 4, bevorzugt 2 : 1 bis 1 : 2, liegt.

In einer Ausführungsform des zweiten Erfindungsgegenstands ist die Zubereitung zur Farbveränderung keratinischer Fasern ein Oxidationsfärbemittel.

In diesem Fall enthält die Alkalisierungszubereitung zusätzlich als farbverändernde Komponente mindestens ein Oxidationsfarbstoffvorprodukt.

Eine Ausführungsform des zweiten Erfindungsgegenstands ist daher eine Zubereitung Farbveränderung keratinischer Fasern, welche dadurch gekennzeichnet ist, dass die Alkalisierungszubereitung zusätzlich mindestens ein Oxidationsfarbstoffvorprodukt enthält.

Bevorzugt ist als Oxidationsfarbstoffvorprodukt mindestens ein Oxidationsfarbstoffvorprodukt vom Entwicklertyp (Entwicklerkomponente), bevorzugt in Kombination mit mindestens einer Oxidationsfarbstoffvorprodukt vom Kupplertyp (Kupplerkomponente), enthalten.
Bevorzugte Oxidationsfarbstoffvorprodukte vom Entwicklertyp sind p-Phenylendiaminderivate. Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diamino-benzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1- yl)propyl]amin, 2-Methoxymethyl-p-phenylendiamin sowie deren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)-ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.
Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-und Pyrazolopyrazol-Derivaten bzw. ihren physiologisch verträglichen Salzen. Bevorzugte PyrimidinDerivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Bevorzugte Pyrazolopyrimidine sind die Verbindungen, die ausgewählt werden unter Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Amino-pyrazolo[1,5-a]pyrimidin-5-ol, 2-(3-Aminopyrazolo[1,5-a]pyrimidin-7-ylamino)ethanol, 2-(7-Amino-pyrazolo[1,5-a]pyrimidin-3-ylamino)ethanol, 2-[(3-Aminopyrazolo[1,5-a]pyrimidin-7-yl)-(2-hydroxyethyl)-amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist. Bevorzugtes Pyrazolopyrazol-Derivat ist 2,3-Diamino-6,7-dihydro-1H,5H-pyrazolo[1,2-a]pyrazol-1-on. Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen. Ganz besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)-pyrazol sowie deren physiologisch verträglichen Salze.

Die Entwicklerkomponenten werden bevorzugt in einer Menge von 0,0001 bis 2,5 Gew.-%, vorzugsweise 0,001 bis 1,5 Gew.-%, jeweils bezogen auf die anwendungsbereite Zubereitung, eingesetzt. Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäße Kupplerkomponenten werden bevorzugt als mindestens eine Verbindung aus einer der folgenden Klassen ausgewählt: m-Aminophenol, o-Aminophenol, m-Diaminobenzol, o-Diaminobenzol und/oder deren Derivate; Naphthalinderivate mit mindestens einer Hydroxygruppe; Dibeziehungsweise Trihydroxybenzol; Pyridinderivate; Pyrimidinderivate; bestimmte Indol-Derivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin); Chinoxalinderivate (beispielsweise 6-Methyl-1,2,3,4-tetrahydrochinoxalin), sowie Gemische aus zwei oder mehreren Verbindungen aus einer oder mehreren dieser Klassen.

Bevorzugte m-Aminophenol-Kupplerkomponenten werden ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen.

Bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen.

Bevorzugte o-Diaminobenzol-Kuppler komponenten werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1- naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxy-pyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und deren physiologisch verträglichen Salzen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxy-pyrimidin, 2,4,6-Trihydroxypyrimidin, 2-Amino-4-methylpyrimidin, 2-Amino-4-hydroxy-6-methyl-pyrimidin und 4,6-Dihydroxy-2-methylpyrimidin und deren physiologisch verträglichen Salzen.

Bevorzugte Indolderivate werden ausgewählt aus mindestens einer Verbindung der Gruppe, die gebildet wird aus 4-Hydroxyindol, 6-Hydroxyindol und 7-Hydroxyindol und deren physiologisch verträglichen Salzen.

Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methytbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2- methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 2,5 Gew.-%, vorzugsweise 0,001 bis 1,0 Gew.-%, jeweils bezogen auf die anwendungsbereite Zubereitung, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Darüber hinaus kann als farbgebende Komponente zusätzlich mindestens ein direktziehender Farbstoff enthalten sein. Dabei handelt sich um Farbstoffe, die direkt auf das Haar aufziehen und keinen oxidativen Prozess zur Ausbildung der Farbe benötigen. Direktziehende Farbstoffe können in anionische, kationische und nichtionische direktziehende Farbstoffe unterteilt werden. Üblicherweise sind es Nitrophenylendiamine, Nitroaminophenole, Azofarbstoffe, Anthrachinone oder Indophenole. Die direktziehenden Farbstoffe werden jeweils bevorzugt in einer Menge von 0,0001 bis 2,0 Gew.-%, bevorzugt von 0,001 bis 1,0 Gew.-%, jeweils bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, HC Blue 16 (Bluequat B), Basic Blue 347, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor® vertrieben werden, sind erfindungsgemäß ebenfalls bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Erfindungsgemäß bevorzugte Farbstoffkombinationen sind solche, die mindestens die Kombination aus Tetrabromphenolblau und Acid Red 92; Tetrabromphenolblau und Acid Red 98; Tetrabromphenolblau und Acid Red 94; Tetrabromphenolblau und Acid Red 87 oder Tetrabromphenolblau und Acid Red 51.

Als farbverändernde Komponente in Aufhellmitteln können schließlich zusätzliche Bleichkraftverstärker, die die Wirkung des Wasserstoffperoxids des Mittels des ersten Erfindungsgegenstands verstärken, eingesetzt werden.

In einer Ausführungsform enthält die erfindungsgemäße eine Alkalisierungszubereitung als farbverändernde Komponente daher einen zusätzlichen Bleichkraftverstärker. Als zusätzliche Bleichkraftverstärker können im Rahmen dieser Erfindung Peroxoverbindungen, weiterhin Verbindungen, die unter Perhydrolysebedingungen aliphatische Peroxocarbonsäuren und/oder substituierte Perbenzoesäure ergeben, Kohlensäurederivate, insbesondere Carbonatsalze bzw. Hydrogencarbonatsalze von Ammonium-, Alkali- oder Erdalkalimetallen, Alkylcarbonate, -carbamate, Silylcarbonate und -carbamate eingesetzt werden.

Vorzugsweise ist der Bleichkraftverstärker ausgewählt aus Ammoniumperoxodisulfat, Alkalimetallperoxodisulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Er findungsgemäß besonders bevorzugt sind Mittel, die als Bleichkraftverstärker mindestens ein anorganisches Salz, ausgewählt aus Peroxomonosulfaten und/oder Peroxodisulfaten, enthalten. Weiter hin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat. Die Peroxoverbindungen sind in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Der Einsatz von Persulfatsalzen bzw. Peroxodisulfatsalzen erfolgt in der Regel wasserfrei und in Form eines gegebenenfalls entstaubten Pulvers, einer Paste oder eines gepressten Formkörpers.

In einer weiteren, bevorzugten Ausführungsform kann dieFarbveränderungszubereitung (FäZ) als Bleichkraftverstärker mindestens ein kationisches Pyridinium-Derivat enthalten. Bevorzugte Verbindungen sind 4-Acyl-Pyridinium-Derivate und 2-Acylpyridinium-Derivate. Insbesondere bevorzugt sind dabei 2-Acetyl-1-methylpyridinium-p-toluolsulfonat und 4-Acetyl-1-methylpyridinium-p-toluolsulfonat. Weiterhin bevorzugte kationische Pyridinium-Derivate sind dabei kationische 3,4-Dihydroisochinolinium-Derivate, insbesondere N-Methyl-3,4-dihydroisochinolinium-p-toluolsulfonat.

Die neben oder anstelle von Peroxoverbindungen eingesetzten Bleichkraftverstärker sind in den erfindungsgemäßen, kosmetischen Zubereitungen bevorzugt in Mengen von 0,05 bis 10 Gew.-%, insbesondere in Mengen von 0,2 bis 5 Gew.-%, jeweils bezogen auf das Gesamtgewicht des anwendungsbereiten Farbveränderungszubereitung, enthalten.

Zur weiteren Steigerung der Aufhellleistung können der erfindungsgemäßen Zusammensetzung zusätzlich als Bleichverstärker mindestens eine gegebenenfalls hydratisierte SiO₂-Verbindung zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder. Bevorzugte gegebenenfalls hydratisierten SiO₂-Verbindungen sind Kieselsäuren, deren Oligomeren und Polymeren sowie deren Salze. Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Erfindungsgemäß bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Insbesondere Metasilicate, die sich gemäß vorstehender Formel durch das Verhältnis zwischen n und der Summe aus m und p von ≤ 1 auszeichnen und sich als kettenförmige polymere Strukturen des Anions [SiO₃]²⁻ auffassen lassen, können bevorzugt eingesetzt werden. Natriummetasilicat der Formel [NaSiO₃]ₓ, ist dabei besonders bevorzugt.

Erfindungsgemäße, anwendungsbereite Zubereitungen sind vorzugsweise wässrige, fließfähige Zubereitungen. Die erfindungsgemäßen Farbveränderungszubereitungen können weiterhin alle für solche Zubereitungen bekannten Wirk-, Zusatz- und Hilfsstoffe enthalten.

Die anwendungsbereiten Farbveränderungszubereitungen als Mischung aus Alkalisierungszubereitung und wasserstoffperoxid-haltigen Mittel können dabei weitere oberflächenaktive Substanzen, ausgewählt aus nichtionischen und kationischen Tensiden, enthalten.

Nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise neben den bereits beschriebenen ethoxylierten Fettalkoholen die Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C₂-C₆-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol LS, Dehydol LT (Cognis) erhältlichen Typen; Polyglycerinester und alkoxylierte Polyglycerinester, wie beispielsweise Poly(3)glycerindiisostearat (Handelsprodukt: Lameform TGI (Henkel)) und Poly(2)glycerinpolyhydroxy-stearat (Handelsprodukt: Dehymuls PGPH (Henkel)); Polyolfettsäureester, wie beispielsweise das Handelsprodukt Hydagen HSP (Cognis) oder Sovermol-Typen (Cognis); höher alkoxylierte, propoxylierte und insbesondere ethoxylierte, Mono-, Di- und Triglyceride mit Alkoxylierungsgrad von größer als 5, wie beispielsweise Glycerinmonolaurat + 20 Ethylenoxid und Glycerinmonostearat + 20 Ethylenoxid; Aminoxide; Hydroxymischether; Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO); Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen in der Alkylkette und 5 bis 30 Ethylenoxid- und/oder Propylenoxid-Einheiten; Alkylpolyglykoside entsprechend der allgemeinen Formel RO-(Z)ₓ, wobei R für Alkyl, Z für Zucker sowie x für die Anzahl der Zuckereinheiten steht.

Zu den nichtionischen Emulgatoren im Sinne der Erfindung zählen weiterhin die Polymerisationsprodukte aus Ethylenoxid und Propylenoxid an gesättigte oder ungesättigte Fettsäureester mehrwertiger Alkohole mit gesättigten oder ungesättigten Fettsäuren; Alkylester von gesättigten oder ungesättigten Fettsäuren oder Alkylphenole und deren Alkoxylate; insbesondere Ethylenglykolether von Fettalkoholen; gemischte Ethylen- und Propylenglykolether mit Fettalkoholen; Fettsäureester an Sorbitan sowie Polyethylenglykol; Ester von nicht hydroxylierten C₆-C₃₀-Alkylmonocarbonäuren mit Polyethylenglykol; und Anlagerungsprodukte von Alkylphenolen an Ethylen- und/oder Propylenoxid.

Erfindungsgemäß bevorzugt sind in anwendungsbereiten Zubereitungen kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride sowie die unter den INCl-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Alkylamidoamine werden üblicherweise durch Amidierung natürlicher oder synthetischer Fettsäuren und Fettsäureschnitte mit Dialkylaminoaminen hergestellt, wie Stearamidopropyl-dimethylamin. Ebenfalls bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex, Dehyquart und Armocare vertrieben. Die Produkte Armocare VGH-70, ein N,N-Bis(2-Palmitoyloxyethyl)dimethylammoniumchlorid, sowie Dehyquart F-75, Dehyquart C-4046, Dehyquart L80 und Dehyquart AU-35 sind Beispiele für solche Esterquats.

Die Tenside sind in den erfindungsgemäß verwendeten Farbveränderungszubereitungen bevorzugt in Mengen von 0,05 bis 25 Gew.-%, bezogen auf die gesamte Farbveränderungszubereitung, enthalten. Mengen von 0,1 bis 15 Gew.-%, insbesondere 1 bis 10 Gew.-%, sind besonders bevorzugt.

Weitere, erfindungsgemäß einsetzbare Wirk-, Hilfs- und Zusatzstoffe sind beispielsweise nichtionische Polymere (wie Vinylpyrrolidinon/Vinylacrylat-Copolymere, Polyvinylpyrrolidinon und Vinylpyrrolidinon/Vinylacetat-Copolymere und Polysiloxane); anionische Polymere (wie Polyacrylsäuren, vernetzte Polyacrylsäuren, Vinylacetat / Crotonsäure-Copolymere, Vinylpyrrolidinon/Vinylacrylat-Copolymere, Vinylacetat / Butylmaleat / Isobornylacrylat-Copolymere, Methylvinylether/Maleinsäureanhydrid-Copolymere und Acrylsäure/Ethylacrylat/N-tert.Butyl-acrylamid-Terpolymere); Verdickungsmittel (wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie Bentonit oder vollsynthetische Hydrokolloide wie z.B. Polyvinylalkohol); Strukturanden (wie Zucker, Maleinsäure und Milchsäure) und Konsistenzgeber (wie Zuckerester, Polyolester oder Polyolalkylether); Proteinhydrolysate (insbesondere Elastin-, Kollagen-, Keratin-, Milcheiweiß-, Sojaprotein- und Weizenproteinhydrolysate, deren Kondensationsprodukte mit Fettsäuren); Parfümöle; Cyclodextrine; Lösungsmittel und -vermittler (wie Ethanol, Isopropanol, Ethylenglykol, Propylenglykol, Glycerin, Dimethylisosorbid und Diethylenglykol); Entschäumer wie Silicone; Farbstoffe und Pigmente zum Anfärben des Mittels; Antischuppenwirkstoffe (wie Piroctone Olamine, Zink Omadine und Climbazol); Lichtschutzmittel (insbesondere derivatisierte Benzophenone, Zimtsäure-Derivate und Triazine); Wirkstoffe (wie Allantoin, Pyrrolidoncarbonsäuren, Cholesterin und deren Salze); weitere Fette und Wachse (wie Bienenwachs, Montanwachs und Paraffine); Quell- und Penetrationsstoffe (wie Glycerin, Propylenglykolmonoethylether, Carbonate, Hydrogencarbonate, Guanidine, Harnstoffe sowie primäre, sekundäre und tertiäre Phosphate); Trübungsmittel (wie Latex, Styrol/PVP- und Styrol/Acrylamid-Copolymere); Perlglanzmittel (wie Ethylenglykolmono- und -distearat sowie PEG-3-distearat); Treibmittel (wie Propan-Butan-Gemische, N₂O, Dimethylether, CO₂ und Luft) sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Farbveränderungszubereitungen besitzen eine Viskosität, die ein leichtes Auftragen und Verteilen auf den zu behandelnden Fasern erlaubt, aber gleichzeitig einen Verbleib am gewünschten Wirkungsort während der Anwendungsdauer garantiert. Die Farbveränderungszubereitungen verfügen somit über gute Anwendungsviskositäten und zeichnen sich dabei durch einen reduzierten Gehalt an polymeren Verdicker aus.

Bevorzugt besitzen die erfindungsgemäßen Farbveränderungszubereitungen daher eine Viskosität von 5 bis 50 Pa·s, bevorzugt 10 bis 20 Pa·s. Die innerhalb dieser Anmeldung genannten Viskositäten wurden jeweils zur besseren Vergleichbarkeit der Messergebnisse mit dem Viskosimeter DV-II+Pro der Firma Brookfield mit der Spindel #5 bei 4 Upm (Umdrehungen pro min) bei RT (22°C) in 590ml Bechergläser, hohe Form, gemessen.

Die Farbveränderungszubereitung wird unmittelbar vor der Anwendung durch Vermischen des Wasserstoffperoxid-haltigen Mittels des ersten Erfindungsgegenstands und der Alkalisierungszubereitung hergestellt. Unmittelbar meint hierbei einen Zeitraum von wenigen Sekunden bis höchstens 10 min. Anschließend wird das Mittel zur Anwendung auf die keratinischen Fasern, insbesondere menschliche Haare, aufgetragen.

Ein weiterer Erfindungsgegenstand ist daher ein Verfahren zur Farbveränderung keratinischer Fasern, insbesondere menschlicher Haare, welches mehrere Verfahrensschritte umfasst:
i) Herstellung einer Zubereitung zur Farbveränderung keratinischer Fasern unmittelbar vor der Anwendung durch Vermischen von mindestens einem Mittel des ersten Erfindungsgegenstands und mindestens einer Alkalisierungszubereitung, enthaltend in einem kosmetisch geeigneten Träger mindestens ein Alkalisierungsmittel und
   zusätzlich
   (i) mindestens einen Fettalkohol und/oder
   (ii) mindestens ein anionisches, amphoteres und/oder zwitterionisches Tensid und/oder
   (iii) mindestens eine Aminosäure und/oder
   (iv) mindestens einen ethoxylierten Fettalkohol und/oder
   (v) mindestens ein kationisches und/oder amphoteres Polymer;
ii) Aufbringen der anwendungsbereiten Zubereitung aus Verfahrensschritt i) auf die zu behandelnden keratinischen Fasern und belassen der Zubereitung für eine Einwirkzeit von 5 bis 60 min auf den Fasern;
iii) Ausspülen der Fasern.

Ein besonderer Vorteil im erfindungsgemäßen Verfahren besteht darin, dass durch die Polymerkombination des wasserstoffperoxid-haltigen Mittels eine homogene Anwendungsmischung mit deutlich geringerem Arbeits- und Zeitaufwand erzielbar ist.

Dies lässt sich beispielsweise dadurch quantifizieren, dass nach Zusammenführen eines Mittel des ersten Erfindungsgegenstands und einer Alkalisierungszubereitung im produktspezifischen Mengenverhältnis in geeigneten Verkaufspackmaterialien, wie einer Flasche, angemischt. Das Vermischen erfolgt üblicherweise durch Schütteln der wiederverschlossenen Flasche. Während des Verschüttelns werden die benötigten "Schüttelvorgänge/ Schläge" der Flasche gezählt, wobei für die erfindungsgemäßen Farbveränderungsmittel signifikant weniger Schüttelvorgänge als bei handelsüblichen Vergleichsprodukten erforderlich sind, um eine homogene Anwendungsmischung zu erhalten.

Ein weiterer Vorteil im erfindungsgemäßen Verfahren besteht darin, dass durch die Polymerkombination des wasserstoffperoxid-haltigen Mittels verbesserte Ausbringung der Anwendungsmischung aus der Applikationsflasche möglich ist.
Hierzu lässt wird dokumentiert, wie viele "Schläge" der Flasche nötig sind, um die Anwendungsmischung aus der Flasche, in der die Anmischung erfolgte, durch die Applikationsspitze analog zur Kundenanwendung zu entnehmen. Es zeigte sich, dass für erfindungsgemäßen Farbveränderungsmittel sind signifikant weniger Aufwand zur Entnahme der Anwendungsmischung als bei handelsüblichen Vergleichsprodukten erforderlich ist, so dass die Anwendung für den Nutzer erheblich erleichtert ist.

Im Falle eines Oxidationsfärbemittels beträgt die bevorzugte Einwirkzeit 5 bis 40 min, bevorzugt 10 bis 30 min. Im Falle von aufhellenden oder bleichenden Farbveränderungsmitteln (Aufhell- bzw. Blondiermittel) liegt die bevorzugte Einwirkzeit bei 30 bis 60 min, bevorzugt bei 40 bis 60 min.

Die Anwendungstemperaturen können in einem Bereich zwischen 15 und 40 °C liegen. Nach der Einwirkungszeit wird das Farbveränderungsmittel durch Ausspülen von dem Haar entfernt. Das Nachwaschen mit einem Shampoo entfällt, wenn ein stark tensidhaltiger Träger verwendet wurde.

Im Rahmen dieses Gegenstandes der Erfindung gelten mutatis mutandis die oben getroffenen Aussagen analog.

Um Instabilitäten bei der Aufbewahrung der einzelnen Mittel vor der Anwendung zu vermeiden, gleichzeitig jedoch die einzelnen Komponenten dem Anwender gemeinsam bereit zu stellen, ist es zweckmäßig, die Zubereitungen getrennt zu verpacken und in einer Umverpackung auszubieten.

Ein weiterer Erfindungsgegenstand ist daher eine Mehrkomponentenverpackungseinheit (Kit-of-Parts) zur Farbveränderung keratinischer Fasern, insbesondere menschlicher Haare, welche mindestens zwei getrennt voneinander konfektionierte Container umfasst, und welche dadurch gekennzeichnet ist,
dass die erste Container ein Mittel des ersten Erfindungsgegenstands enthält und
dass die zweite Container eine Alkalisierungszubereitung, enthaltend in einem kosmetisch geeigneten Träger mindestens ein Alkalisierungsmittel und mindestens einen Fettalkohol, enthält.

Die Zubereitungen der Mehrkomponentenverpackungseinheit sind in getrennt voneinander konfektionierten Containern enthalten. Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff.

Es kann erfindungsgemäß vorteilhaft sein, wenn die erfindungsgemäße Mehrkomponentenverpackungseinheit mindestens ein weiteres Haarbehandlungsmittel in einem getrennten Container enthält, insbesondere ein Konditioniermittel. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Zur verbesserten Durchmischung ist vorteilhaft, wenn der Container, welcher ein Mittel des ersten Erfindungsgegenstands enthält, eine wieder-verschließbare Öffnung, wie beispielsweise einen Schnapp- oder einen Schraubverschluss, besitzt. Dies ermöglicht die erleichterte Zugabe des Alkalisierungsmittel aus dem zweiten Container, welcher seinerseits bevorzugt in Form eines Tütchens oder Sachets im Falle von wasserfreien, insbesondere pulverförmigen Farbveränderungsmitteln, oder in Form einer Tube im Falle von fließfähigen Farbveränderungsmitteln vorliegt.

Hinsichtlich der weiteren bevorzugten Ausführungsführungsformen der Mehrkomponentenverpackungseinheit gelten mutatis mutandis die obigen Ausführungen der vorangehenden Erfindungsgegenstände.

Durch die Kombination der polymeren Verdicker in den wasserstoffperoxid-haltigen Mitteln lässt sich die Einsatzmenge an polymerem Verdickungsmittel in den Farbveränderungszubereitungen in vorteilhafter Weise reduzieren. Dadurch lassen sich einerseits Rohstoffmengen verringern und andererseits die durch hohe Menge an Verdickungsmittel verursachten Probleme im Herstellungsverfahren minimieren.

Schließlich verbessern sich die Anwendungseigenschaften der Farbveränderungszubereitungen gegenüber handelsüblichen Produkten.

Ein weiterer Gegenstand der vorliegenden Erfindung ist daher die Verwendung eines wasserstoffperoxid-haltiges Mittels des ersten Erfindungsgegenstandes zur Verbesserung der Anwendungseigenschaften einer Zubereitung zur Farbveränderung keratinischer Fasern.

Die verbesserten Anwendungseigenschaften sind dabei einerseits die erleichtere Mischbarkeit des Mittels des ersten Erfindungsgegenstandes mit der Alkalisierungszubereitung und damit eine schnellere, effizientere und anwenderfreundlichere Herstellung einer homogenen Zubereitung zur Farbveränderung keratinischer Fasern, insbesondere menschlicher Haare.

Andererseits zeigen sich die verbesserten Anwendungseigenschaften in einer verbesserten Ausbringung der anwendungsbereiten Zubereitung zur Farbveränderung keratinischer Fasern.

Schließlich ist durch die Verwendung eines wasserstoffperoxid-haltiges Mittels des ersten Erfindungsgegenstandes eine Verringerung des Polymergehalts bei gleichbleibenden oder verbesserten Viskositäten der anwendungsbereiten Farbveränderungszubereitung möglich.

Im Rahmen dieses Gegenstandes der Erfindung gelten mutatis mutandis die oben getroffenen Aussagen analog.

### Beispiele

### 1) Färbecremes - Alkalisierungszubereitungen (Mengenangaben in Gew.-%)

| **Rohstoffe** | **F1** | **F2** | **F3** | **F4** |
|---|---|---|---|---|
| Cetearyl Alcohol | 6,60 | 6,60 | 11,00 | 8,70 |
| Ethanolamine | -- | -- | 6,97 | 5,47 |
| Ammonium Hydroxide | 3,34 | 3,34 | -- | -- |
| Coconut Alcohol | 2,40 | 2,40 | 5,40 | 4,02 |
| Ammonium Chloride | -- | -- | 3,00 | -- |
| Sodium Laureth-6 Carboxylate | 2,10 | 2,10 | -- | -- |
| Sodium Myreth Sulfate | 1,96 | 1,96 | -- | -- |
| Sodium Laureth Sulfate | -- | -- | 0,81 | 0,81 |
| Disodium Cocoamphodipropionate | -- | -- | 0,80 | 0,80 |
| Cocamidopropyl Betaine | -- | -- | 0,61 | 0,61 |
| Serine | 1,00 | -- | -- | -- |
| Arginine | -- | -- | 1,00 | 1,00 |
| Ammonium Sulfate | 0,93 | 0,29 | -- | 2,00 |
| Acrylamidopropyltrimonium Chloride/Acrvlates Copolymer | 0,74 | 0,73 | -- | -- |
| Polvquaternium-22 | -- | -- | 0,62 | 0,62 |
| Xanthan | -- | -- | 0,18 | 0,13 |
| Coco-Glucoside | 0,67 | 0,67 | -- | -- |
| Ceteareth-12 | 0,60 | 0,60 | -- | -- |
| Ceteareth-20 | 0,60 | 0,60 | 0,45 | 0,34 |
| Ceteareth-30 | -- | -- | 0,45 | 0,33 |
| Ceteareth-50 | -- | -- | 1,80 | 1,34 |
| Glyceryl Oleate | 0,56 | 0,56 | 0,20 | -- |
| Sodium Sulfite | 0,40 | 0,40 | -- | 0,20 |
| Sodium Silicate | 0,19 | 0,19 | 0,19 | 0,19 |
| Etidronic Acid | 0,12 | 0,12 | 0,12 | 0,12 |
| Ascorbic Acid | 0,10 | 0,10 | -- | |
| Citric Acid | 0,10 | 0,10 | 0,10 | 0,10 |
| Sodium Hydroxide | 0,10 | -- | -- | -- |
| Sodium Chloride | 0,05 | 0,05 | 0,11 | 0,11 |
| Propylene Glvcol | -- | -- | 0,90 | 0,67 |
| Toluene-2,5-Diamine Sulfate | 0,11 | 1,18 | 3,00 | 0,84 |
| 1,3-Bis-(2,4-Diaminophenoxy) Propane HCl | -- | 0,44 | 0,244 | -- |
| 2,7-Naphthalenediol | -- | 0,29 | -- | -- |
| 4-Chlororesorcinol | 0,03 | -- | -- | -- |
| Resorcinol | 0,03 | 1,66 | 0,87 | 0,52 |
| 2-Methylresorcinol | 0,01 | -- | -- | 0,02 |
| 3-Aminophenol | 0,002 | -- | 0,47 | 0,24 |
| 6-Methoxy-2-Methylamino-3-Aminopyridine HCl | -- | -- | 0,15 | -- |
| 4-Amino-3-methylphenol | -- | -- | -- | 0,67 |
| 5-Amino-2-methylphenol | -- | -- | -- | 0,23 |
| 2-Amino-6-chloro-4-nitrophenol | -- | -- | -- | 0,18 |
| 2-Amino-3-hydroxypyridine | -- | -- | -- | 0,14 |
| 1-Naphthol | -- | -- | -- | 0,05 |
| 4-Amino-3-nitrophenol | -- | -- | -- | 0,05 |
| Parfum | qs | qs | qs | qs |
| Aqua | Ad 100 | | | |

Die Fettbasis wurde zusammen bei 80 °C aufgeschmolzen und mit einem Teil der Wassermenge dispergiert. Anschließend wurden die restlichen Rezepturbestandteile unter Rühren der Reihe nach eingearbeitet. Es wurde mit Wasser auf 100 Gew.-% aufgefüllt und die Formulierung kalt gerührt.

### 2) Wasserstoffperoxid-haltige Mittel (Mengenangaben in Gew.-%)

| **Rohstoff** | **V1** | **E1** | **V2** | **E2** |
|---|---|---|---|---|
| Kaliumhydroxid | 0,12 | 0,12 | -- | -- |
| Natriumhydroxid | -- | -- | 0,33 | 0,33 |
| 2,6-Dicarboxypyridine | 0,10 | 0,10 | 0,10 | 0,10 |
| Dinatriumpyrophosphat | 0,03 | 0,03 | 0,03 | 0,03 |
| Etidronic Acid | 0,15 | 0,15 | 0,90 | 0,90 |
| Sodium Laureth Sulfate | 0,54 | 0,33 | 0,54 | 0,54 |
| Luvigel Star | -- | 10,00 | -- | 0,85 |
| Aculyn 33A | 15,00 | 1,20 | 2,50 | 0,11 |
| Wasserstoffperoxid | 5,93 | 5,93 | 7,42 | 7,42 |
| Wasser, vollentsalzt | Ad 100 | Ad 100 | Ad 100 | Ad 100 |

Rohstoffe: Aculyn 33A (ca. 28% Aktivsubstanz; INCI-Bezeichnung: Acrylates Copolymer, Aqua; Rohm & Haas); Luvigel Star (ca. 20% Aktivsubstanz; INCI-Bezeichnung: Polyurethan-39; BASF).

### 3) Anwendungsmischungen:

Die Färbecremes F1 und F2 wurden jeweils mit den Mitteln E1 und V1 sowie die Färbecremes F3 und F4 jeweils mit den Mitteln E2 und V2 bei Raumtemperatur im Gewichtsverhältnis von 1:1 miteinander versetzt und innig vermischt.

Dabei wurden folgende Mischviskositäten erhalten:

| | **Farbveränderungszubereitung** | **Polymergehalt*** | **Mischviskosität**** |
|---|---|---|---|
| #1 | F1 + V1 (nicht erfindungsgemäß) | 2,1% PAc | 25000 |
| #2 | F1 + E1 (erfindungsgemäß) | 0,1% PAc + 1,0 PUr | 33000 |
| #3 | F2 + V1 (nicht erfindungsgemäß) | 2,1% PAc | 20000 |
| #4 | F2 + E1 (erfindungsgemäß) | 0,1% PAc + 1,0 PUr | 20000 |
| #5 | F3 + V2 (nicht erfindungsgemäß) | 0,35% PAc | 21000 |
| #6 | F3 + E2 (erfindungsgemäß) | 0,015% PAc + 0,085% PUr | 23000 |
| #7 | F4 + V2 (nicht erfindungsgemäß) | 0,35% PAc | 14000 |
| #8 | F4 + E2 (erfindungsgemäß) | 0,015% PAc + 0,085% PUr | 15000 |

| | | | |
|---|---|---|---|
| * in Gew.-% der Anwendungsmischung; PAc = Acrylates Copolymer; PUr = Polyurethan-39 ** in mPa·s, gemessen mit einem Brookfield-Viskosimeter DV-II+Pro mit Spindel #5 bei 4 Upm bei RT (22°C) in 590 ml Bechergläser, hohe Form. | | | |

Die erfindungsgemäßen, anwendungsbereiten Farbveränderungszubereitungen zeichnen sich dabei gegenüber den Vergleichsmitteln durch eine gleichbleibende oder verbesserte Viskosität aus, obwohl die absolute Polymer-Menge signifikant verringert war.

## Patentansprüche

1. Mittel, enthaltend in einem kosmetischen geeigneten Träger mindestens Wasserstoffperoxid, mindestens ein Homo- oder Copolymer von Acrylsäure und/oder Methacrylsäure und mindestens ein Polyurethan, **dadurch gekennzeichnet, dass**
- das Polyurethan ein Polykondensat aus Polyethylenglycol(en), Diisocyanat(en) und gegebenenfalls ethoxylierten Fettalkohol(en) ist und
- das Mittel als Homo- und/oder Copolymer von Acrylsäure und/oder Methacrylsäure mindestens ein Copolymer aus Ethylacrylat sowie Methacrylsäure und/oder Acrylsäure enthält und
- das Polyurethan in einem Gewichtsanteil von 0,05 bis 3,0 Gew.-%, jeweils bezogen auf das Gesamtgewicht des Mittels, enthalten ist.

2. Mittel nach Anspruch 1, **dadurch gekennzeichnet, dass** das Polyurethan ein Polykondensat aus Polyethylenglycol mit 50 bis 250 Einheiten Ethylenglycol (PEG-50 bis PEG-250), einem Diisocyanat und ethoxylierten C₁₂-C₂₀-Fettalkoholen mit einem Ethoxylierungsgrad von 6 bis 25 ist.

3. Mittel nach Anspruch 1 oder 2, **dadurch gekennzeichnet, dass** das Polyurethan ein Polykondensat aus PEG-140, Hexamethylendiisocyanat und einem Gemisch aus C₁₂-C₁₄-Pareth-10, C₁₆-C₁₈-Pareth-11 und C₁₈-C₂₀-Pareth-11 ist.

4. Mittel nach einem der Ansprüche 1 bis 3, **dadurch gekennzeichnet, dass** das Polyurethan in einem Gewichtsanteil von 0,1 bis 2,5 Gew.-%, jeweils bezogen auf das Gesamtgewicht des Mittels, enthalten ist.

5. Mittel nach einem der Ansprüche 1 bis 4, **dadurch gekennzeichnet, dass** das/die Homo- und/oder Copolymer(e) von Acrylsäure und/oder Methacrylsäure in einem Gewichtsanteil von 0,001 bis 2,0 Gew.-%, bevorzugt 0,005 bis 1,0 Gew.-% und insbesondere von 0,01 bis 0,5 Gew.-%, jeweils bezogen auf das Gesamtgewicht des Mittels, enthalten ist.

6. Zubereitung zur Farbveränderung keratinischer Fasern, insbesondere menschlicher Haare, **dadurch gekennzeichnet, dass** die Zubereitung unmittelbar vor der Anwendung durch Vermischen von mindestens einem Mittel nach einem der Ansprüche 1 bis 5 und mindestens einer Alkalisierungszubereitung, enthaltend in einem kosmetisch geeigneten Träger mindestens ein Alkalisierungsmittel und zusätzlich
(i) mindestens einen Fettalkohol und/oder
(ii) mindestens ein anionisches, amphoteres und/oder zwitterionisches Tensid und/oder
(iii) mindestens eine Aminosäure und/oder
(iv) mindestens einen ethoxylierten Fettalkohol und/oder
(v) mindestens ein kationisches und/oder amphoteres Polymer,
hergestellt wird.

7. Zubereitung nach Anspruch 6, **dadurch gekennzeichnet, dass** die Alkalisierungszubereitung zusätzlich mindestens ein Oxidationsfarbstoffvorprodukt enthält.

8. Verfahren zur Farbveränderung keratinischer Fasern, insbesondere menschlicher Haare, umfassend mehrere Verfahrensschritte:
i) Herstellung einer Zubereitung zur Farbveränderung keratinischer Fasern unmittelbar vor der Anwendung durch Vermischen von mindestens einem Mittel nach einem der Ansprüche 1 bis 5 und mindestens einer Alkalisierungszubereitung, enthaltend in einem kosmetisch geeigneten Träger mindestens ein Alkalisierungsmittel und zusätzlich
(i) mindestens einen Fettalkohol und/oder
(ii) mindestens ein anionisches, amphoteres und/oder zwitterionisches Tensid und/oder
(iii) mindestens eine Aminosäure und/oder
(iv) mindestens einen ethoxylierten Fettalkohol und/oder
(v) mindestens ein kationisches und/oder amphoteres Polymer;
ii) Aufbringen der anwendungsbereiten Zubereitung aus Verfahrensschritt i) auf die zu behandelnden keratinischen Fasern und belassen der Zubereitung für eine Einwirkzeit von 5 bis 60 min auf den Fasern;
iii) Ausspülen der Fasern.

9. Mehrkomponentenverpackungseinheit (Kit-of-Parts) zur Farbveränderung keratinischer Fasern, insbesondere menschlicher Haare, umfassend mindestens zwei getrennt voneinander konfektionierte Komponenten, **dadurch gekennzeichnet,**
**dass** die erste Komponente ein Mittel nach einem der Ansprüche 1 bis 5 darstellt und
**dass** die zweite Komponente eine Alkalisierungszubereitung nach einem der Ansprüche 6 oder 7 darstellt.

10. Verwendung eines Mittels nach einem der Ansprüche 1 bis 5 zur Verbesserung der Anwendungseigenschaften einer Zubereitung zur Farbveränderung keratinischer Fasern.

## Claims

1. An agent, containing, in a cosmetically suitable carrier, at least hydrogen peroxide, at least one homopolymer or copolymer of acrylic acid and/or methacrylic acid, and at least one polyurethane, **characterized in that**
- the polyurethane is a polycondensate of polyethylene glycol(s), diisocyanate(s) and optionally ethoxylated fatty alcohol(s), and
- the agent contains, as the homopolymer and/or copolymer of acrylic acid and/or methacrylic acid, at least one copolymer of ethyl acrylate as well as methacrylic acid and/or acrylic acid, and
- the polyurethane is contained in a weight proportion of from 0.05 to 3.0 wt.%, based in each case on the total weight of the agent.

2. The agent according to claim 1, **characterized in that** the polyurethane is a polycondensate of polyethylene glycol having from 50 to 250 ethylene glycol units (from PEG-50 to PEG-250), a diisocyanate, and ethoxylated C₁₂-C₂₀ fatty alcohols having a degree of ethoxylation of from 6 to 25.

3. The agent according to either claim 1 or claim 2, **characterized in that** the polyurethane is a polycondensate of PEG-140, hexamethylene diisocyanate, and a mixture of C₁₂-C₁₄ pareth-10, C₁₆-C₁₈ pareth-11 and C₁₈-C₂₀ pareth-11.

4. The agent according to one of claims 1 to 3, **characterized in that** the polyurethane is contained in a weight proportion of from 0.1 to 2.5 wt.%, based in each case on the total weight of the agent.

5. The agent according to one of claims 1 to 4, **characterized in that** the homopolymer(s) and/or copolymer(s) of acrylic acid and/or methacrylic acid is/are contained in a weight proportion of from 0.001 to 2.0 wt.%, preferably of from 0.005 to 1.0 wt.%, and in particular of from 0.01 to 0.5 wt.%, based in each case on the total weight of the agent.

6. A preparation for modifying the color of keratin fibers, in particular human hair, **characterized in that** the preparation is prepared immediately before use by mixing at least one agent according to one of claims 1 to 5 and at least one alkalizing preparation containing, in a cosmetically suitable carrier, at least one alkalizing agent and additionally
(i) at least one fatty alcohol, and/or
(ii) at least one anionic, amphoteric and/or zwitterionic surfactant, and/or
(iii)at least one amino acid, and/or
(iv)at least one ethoxylated fatty alcohol, and/or
(v) at least one cationic and/or amphoteric polymer.

7. The preparation according to claim 6, **characterized in that** the alkalizing preparation additionally contains at least one oxidation dye precursor.

8. A method for modifying the color of keratin fibers, in particular human hair, comprising a plurality of method steps:
i) preparing a preparation for modifying the color of keratin fibers immediately before use by mixing at least one agent according to one of claims 1 to 5 and at least one alkalizing preparation containing, in a cosmetically suitable carrier, at least one alkalizing agent and additionally
(i) at least one fatty alcohol, and/or
(ii) at least one anionic, amphoteric and/or zwitterionic surfactant, and/or (iii)at least one amino acid, and/or
(iv)at least one ethoxylated fatty alcohol, and/or
(v) at least one cationic and/or amphoteric polymer;
ii) applying the ready-to-use preparation of method step i) to the keratin fibers to be treated, and leaving the preparation on the fibers for a contact time of from 5 to 60 minutes;
iii) rinsing the fibers.

9. A multicomponent packaging unit (kit of parts) for modifying the color of keratin fibers, in particular human hair, comprising at least two components that are packaged separately from one another, **characterized in that**
the first component is an agent according to one of claims 1 to 5, and **in that** the second component is an alkalizing preparation according to either claim 6 or claim 7.

10. The use of an agent according to one of claims 1 to 5 for improving the application properties of a preparation for modifying keratin fibers.

## Revendications

1. Agent contenant, dans un support cosmétique adapté, au moins du peroxyde d'hydrogène, au moins un homopolymère ou copolymère d'acide acrylique et/ou d'acide méthacrylique et au moins un polyuréthane, **caractérisé en ce que**
- le polyuréthane est un polycondensat de polyéthylène glycol(s), diisocyanate(s) et éventuellement alcool(s) gras éthoxylé(s) et
- l'homopolymère et/ou copolymère d'acide acrylique et/ou d'acide méthacrylique que l'agent contient est au moins un copolymère d'acrylate d'éthyle ainsi que d'acide méthacrylique et/ou d'acide acrylique et
- le polyuréthane est présent dans une proportion en poids de 0,05 à 3,0 % en poids, par rapport au poids total de l'agent respectivement.

2. Agent selon la revendication 1, **caractérisé en ce que** le polyuréthane est un polycondensat de polyéthylène glycol comportant 50 à 250 unités d'éthylène glycol (PEG-50 à PEG-250), d'un diisocyanate, et d'alcools gras en C₁₂-C₂₀ éthoxylés à un degré d'éthoxylation de 6 à 25.

3. Agent selon la revendication 1 ou 2, **caractérisé en ce que** le polyuréthane est un polycondensat de PEG-140, d'hexaméthylène diisocyanate et d'un mélange de C₁₂-C₁₄ Pareth-10, C₁₆-C₁₈ Pareth-11 et C₁₈-C₂₀ Pareth-11.

4. Agent selon l'une quelconque des revendications 1 à 3, **caractérisé en ce que** le polyuréthane est présent dans une proportion en poids de 0,1 à 2,5 % en poids, par rapport au poids total de l'agent respectivement.

5. Agent selon l'une quelconque des revendications 1 à 4, **caractérisé en ce que** l'/les homopolymère(s) et/ou copolymère(s) d'acide acrylique et/ou d'acide méthacrylique est présent dans une proportion en poids de 0,001 à 2,0 % en poids, de préférence 0,005 à 1,0 % en poids et en particulier de 0,01 à 0,5 % en poids, par rapport au poids total de l'agent respectivement.

6. Préparation de coloration de fibres kératiniques, notamment de cheveux humains, **caractérisée en ce que** la préparation est fabriquée juste avant l'application par le mélange d'au moins un agent selon une des revendications 1 à 5 et d'au moins une préparation d'alcalinisation, contenant dans un support cosmétiquement adapté au moins un agent d'alcalinisation ainsi que
(i) au moins un alcool gras et/ou
(ii) au moins un tensioactif anionique, amphotère et/ou zwitterionique et/ou
(iii) au moins un acide aminé et/ou
(iv) au moins un alcool gras éthoxylé et/ou
(v) au moins un polymère cationique et/ou amphotère.

7. Préparation selon la revendication 6, **caractérisée en ce que** la préparation d'alcalinisation contient en plus au moins un précurseur de colorant d'oxydation.

8. Procédé de coloration de fibres kératiniques, notamment de cheveux humains, comprenant plusieurs étapes :
i) Fabriquer une préparation de coloration de fibres kératiniques juste avant l'application par le mélange d'au moins un agent selon une des revendications 1 à 5 et d'au moins une préparation d'alcalinisation, contenant dans un support cosmétiquement adapté au moins un agent d'alcalinisation ainsi que
(i) au moins un alcool gras et/ou
(ii) au moins un tensioactif anionique, amphotère et/ou zwitterionique et/ou
(iii) au moins un acide aminé et/ou
(iv) au moins un alcool gras éthoxylé et/ou
(v) au moins un polymère cationique et/ou amphotère ;
ii) Poser la préparation prête à l'application issue de l'étape i) sur les fibres kératiniques à traiter et laisser la préparation sur les fibres pendant une durée d'action de 5 à 60 min ;
iii) Rincer les fibres.

9. Unité d'emballage multi-composant (Kit-of-Parts) pour la coloration de fibres kératiniques, notamment de cheveux humains, comprenant au moins deux composants conditionnés séparément l'un de l'autre, **caractérisée en ce que**
le premier composant est un agent selon une des revendications 1 à 5 et
le deuxième composant est une préparation d'alcalinisation selon une des revendications 6 ou 7.

10. Utilisation d'un agent selon l'une quelconque des revendications 1 à 5 pour améliorer les propriétés d'application d'une préparation de coloration de fibres kératiniques.
